# EUROPEAN PATENT APPLICATION

(11) **EP 4 282 362 A1**
(43) Date of publication of application: **29.11.2023**
(21) Application number: 22382499.6
(22) Date of filing: 25.05.2022
(51) Int. Cl.: A61B 18/04, A61B 18/14, A61B 17/29

(54) **SURGICAL INSTRUMENT FOR CAPTURING AND FRAGMENTING SEVERED PROSTATE TISSUE**

(71) Applicant: Fundació Institut d'Investigació en Ciències de la Salut Germans Trias i Pujol (IGTP), 08916 Badalona (ES)
(72) Inventor: BUISÁN RUEDA, Óscar, 08916 Badalona (ES)
(74) Representative: Hoffmann Eitle

(57) **Abstract**

The invention refers to a tissue morcellator. The morcellator comprises: an elongate body configured to slidably fit inside a cannula, an ablation element located at a distal portion of the elongate body, a fluid evacuation conduit for fluidly communicating the distal portion of the elongate body to a proximal portion of the morcellator, and at least a pair of clamping elements proximal the ablation element and movable between an open configuration and a closed configuration, configured to bring tissue in contact with the ablation element when moved from the open configuration towards the closed configuration. The surgical instrument facilitates the process of extracting severed prostate tissue after prostate enucleation, as to reduce surgery time and reduce the risk of accidentally damaging the prostate.

## Description

### Field and object of the invention

The present invention refers to surgical instruments for capturing and fragmenting residual slices of prostate tissue.

An object of the invention is to provide a surgical instrument of the above type, that facilitates and speeds up the process of extracting severed prostate tissue after prostate enucleation, as to reduce surgery time and reduce the risk of accidentally damaging the prostate.

### Background of the invention

Bipolar electrodes are commonly used for prostate enucleation during which prostate is sliced by a bipolar RF energy in a conductive saline solution. Bipolar electrosurgical devices have the advantage over monopolar devices, that the return current path does not flow through the patient, thus, in bipolar electrosurgical devices, both the active and return electrode are typically closely exposed so that both electrodes may contact tissue, thereby providing a return current path from the active to the return electrode only through the target tissue. This results in highly localized tissue damage, and minimal thermal damage to peripheral tissue.

An example of a bipolar ablation electrode is described in the European patent application EP 1072230 A1.

After prostate enucleation by means of a bipolar electrode, the pieces of severed prostate tissue are generally too large to be extracted thorough a cannula, so these pieces have to be captured and fragmented by means of another surgical instrument specially adapted for this purpose. Currently, the so-called "morcellator" instruments are used for that task, that are generally based on grinding devices. U.S patent US 5569284 describes an example of a morcellator device.

Mechanical morcellators have the drawback that the process for capturing and fragmenting all excised prostate tissue is time consuming, because the surgeon has to catch prostate slices to bring them in contact with a cutting member, so that morcellators require highly skilled hands to be used safely to avoid damaging the bladder.

Therefore, with the existing technology the surgical process of extracting prostate tissue is time-consuming and risky for patients.

### Summary of the invention

The present invention is defined in the attached independent claim, and satisfactorily solves the drawbacks of the prior art, by providing a morcellator suitable for capturing and fragmenting residual slices of prostate tissue that combines at least two clamping elements, and an ablation element to carry out the above process, so that it does not rely on grinding mechanical devices.

More specifically, an aspect of the invention refers to a morcellator of the above-mentioned type, that comprises elongate body configured to slidably fit inside a cannula.

The instrument additionally comprises an ablation element located at a distal portion of the elongate body, an fluid evacuation conduit for fluidly communicating the distal portion of the elongate body to a proximal portion of the morcellator and connectable to a negative pressure source at a proximal end of the fluid evacuation conduit, and at least two clamping elements proximal the ablation element and movable between an open configuration and a closed configuration, configured to bring tissue in contact with the ablation element when moved from the open configuration towards the closed configuration.

With this combination of extendable and retractable clamping elements, the tissue is both clamped and brought into contact with the ablation element, allowing improved controllability of the tissue morcellation process. Using the morcellator of the invention, for a surgeon it is very convenient to easily capture floating slices of prostate, and as the clamping elements are retracted the slices are cut by the ablation element, thereby simplifying the extraction process.

In a preferred embodiment, the elongate body is a tubular body comprising the fluid extraction evacuation conduit. Moreover, the ablation element is preferably located in the tubular body and exposed via an aperture in the tubular body, wherein the aperture is fluidly connected to a distal portion of the evacuation conduit. Advantageously, in such embodiments the ablation element and evacuation conduit are provided in the same tubular body and so the fluid communication between the aperture, where tissue is ablated, and the fluid evacuation conduit, is maintained.

In a more preferred embodiment, the aperture comprises a aperture having a lateral portion in a sidewall of the tubular body, and the at least two clamping elements are configured to move towards the aperture from the open configuration to the closed configuration. Advantageously, the provision of an aperture with a lateral portion allows for a larger area of tissue to be ablated more efficiently. clamping elementsclamping elements

In a preferred embodiment, the at least two clamping elements are pivotably mounted to the morcellator. More preferably, the at least two clamping elements are pivotably mounted to the elongate body which provides a more compact morcellator. clamping elementsclamping elementsclamping elements

More preferably, one or more of the clamping elements are received in one or more recesses of the morcellator in the closed position, preferably wherein the one or more clamping elements are fully received within the one or more recesses in the closed position. This provides a smoother surface when the elongate body is exposed but the clamping elements are retracted, allowing for movement such as rotation of the morcellator whilst reducing damages to healthy tissues by catching on an exposed part of a clamping element.

In another preferred embodiment, the ablation element comprises a plasma ablation element. Preferably, the plasma ablation element comprises a bipolar electrode, comprising at least one feed electrode and at least one return electrode. More preferably, the feed electrode is made of a biocompatible high temperature metal or metal alloy. Even more preferably, the feed electrode is made of: tungsten, tungsten-rhenium, platinum-iridium, titanium, or stainless steel. Preferably, the plasma ablation element is configured to be longitudinally displaceable relative to the elongate body.

In some embodiments, the elongate body has an atraumatic tip, preferably wherein the elongate body has a rounded cross-sectional shape, to reduce the risk of trauma to tissue when the morcellator is being positioned.

In a preferred embodiment, the morcellator further comprises an insulating element electrically insulating a portion of feed electrode from the return electrode along at least a portion of the morcellator, wherein the feed electrode and the return electrode are exposed at the distal portion of the elongate body. Preferably the insulating element is provided along the entire length of the morcellator apart from the distal portion of the elongate body This reduces the risk of electrical current passing between the electrodes away from the target ablation site.

Preferably the tissue morcellator also comprises the negative pressure source.

According to a second aspect of the invention, there is provided a tissue morcellating system comprising a tissue morcellator according to the first aspect and a cannula suitable to be inserted through a patient's urethra, and sized to slidably receive the tissue morcellator. Preferably the cannula is a metallic tube. In some embodiments the

### Brief description of the drawings

Preferred embodiments of the invention, by way of example only and to enable a better understanding of the present invention, are henceforth described with reference to the accompanying drawings, wherein:
Figure 1.- shows a perspective view of a preferred embodiment of a morcellator slidably extended from a cannula, with the clamping elements in their open position (A); with the clamping elements in their closed position (B) and a see-through view of the morcellator inside the cannula (C).
Figure 2.- shows a perspective view of the instrument of Figure 1 slidably retracted inside the cannula (A) and a see-through view of the morcellator inside the cannula (B).
Figure 3.- shows a perspective view of another morcellator according to one or more embodiments.
Figure 4.- shows a perspective view of a morcellator according to an alternative embodiment.
Figure 5.- shows a perspective view of another morcellator according to an alternative embodiment.
Figure 6.- shows a schematic diagram of a pair of clamping elements according to one or more preferred embodiments (A) in closed configuration and (B) in open configuration.

### Detailed description

Throughout this disclosure the term "ablation element" may refer to any device suitable for ablating tissue, and includes but is not limited to any plasma ablation, laser ablation, or mechanical ablation element.

Throughout this disclosure the term "clamping element" may refer to any element suitable for capturing tissue between two or more of the clamping elements. The clamping may be generated by more than two clamping elements. The clamping elements may include, for example, arms, hooks, bars or any other element which can be used to clamp tissue between the elements. The clamping elements may take any suitable shape and may be made of any suitable material.

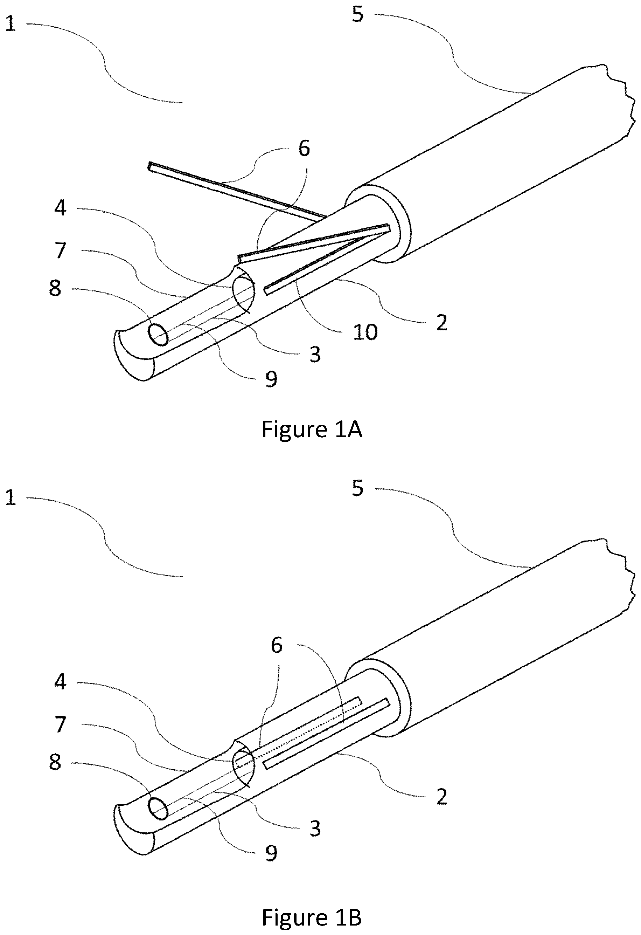

**Figure 1A** shows a preferred implementation of a morcellator (1) according to the invention, that comprises an elongate body (2) configured to slidably fit inside a cannula (5) suitable to be inserted through a patient's urethra.

The morcellator (1) includes an ablation element (8) located at a distal portion of the elongate body (2) and a fluid evacuation conduit (4) for fluidly communicating the distal portion of the elongate body (2) to a proximal portion of the morcellator. The fluid evacuation conduit is connectable to a negative pressure source at a proximal end of the fluid evacuation conduit, which in use remains outside of the patient.

The morcellator (1) further includes at least two clamping elements (6) for capturing tissue. The at least two clamping elements (6) are provided proximal the ablation element (8) and movable between an open configuration and a closed configuration, configured to clamp tissue and bring the clamped tissue in contact with the ablation element when moved from the open configuration towards the closed configuration.

In any of the embodiments disclosed herein, the distal tip of one or more of the at least two clamping elements (6) preferably comprise a hook in order to improve the grip on the captured tissue. This may be achieved by a tooth-shaped tip or by any other inwardly facing protrusion in the direction of movement from the open configuration to the closed configuration.

The morcellator (1) is actuatable to move the plurality of clamping elements (6) between the open configuration shown in Figure 1A and the closed configuration shown in Figure 1B, via any suitable mechanism such as those disclosed in further detail herein. In use, the cannula (5) is inserted through a lumen of the body, for example the urethra, until the distal end is correctly placed at the target site. The distal portion of the morcellator (1) is positioned at the target site by sliding the morcellator (1) through the cannula (5). The clamping elements (6) are moved to the open position. When the clamping elements (6) are moved from the open position shown in Figure 1A to the closed position shown in Figure 1B. the clamping elements (6) move both towards each other and towards the ablation element (8). The movement of the clamping elements (6) towards each other clamping elements any tissue situated between the clamping elements (6). The movement of the clamping elements (6) towards the ablation element (8) brings the clamped tissue into contact with the ablation element (8). Accordingly, when the ablation element (8) is active, the clamped tissue is ablated. The clamping elements (6) may be moved between the open and closed configurations as many times as necessary to ablate the desired amount of tissue.

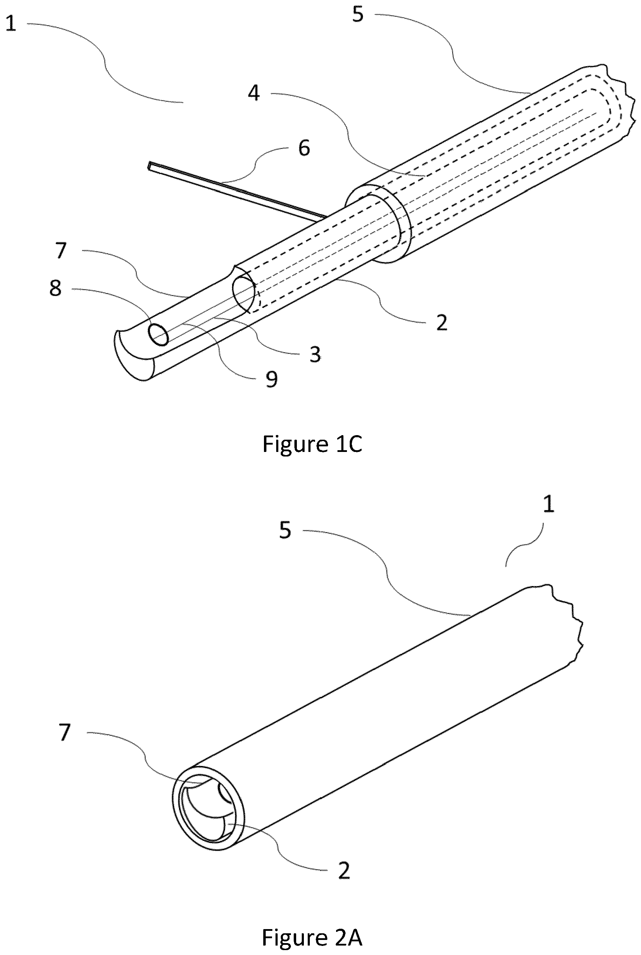

Furthermore, as shown in Figure 1C, the fluid evacuation conduit (4) provides a route for the ablated tissue and any fluids away from the distal portion of the morcellator (1) and towards a proximal end of the morcellator (1) outside of the patient. For example, a negative pressure source may be connected to the proximal end of the fluid evacuation conduit outside of the patient to generate a suction at the distal portion of the morcellator (1) proximal to the ablation element (8).

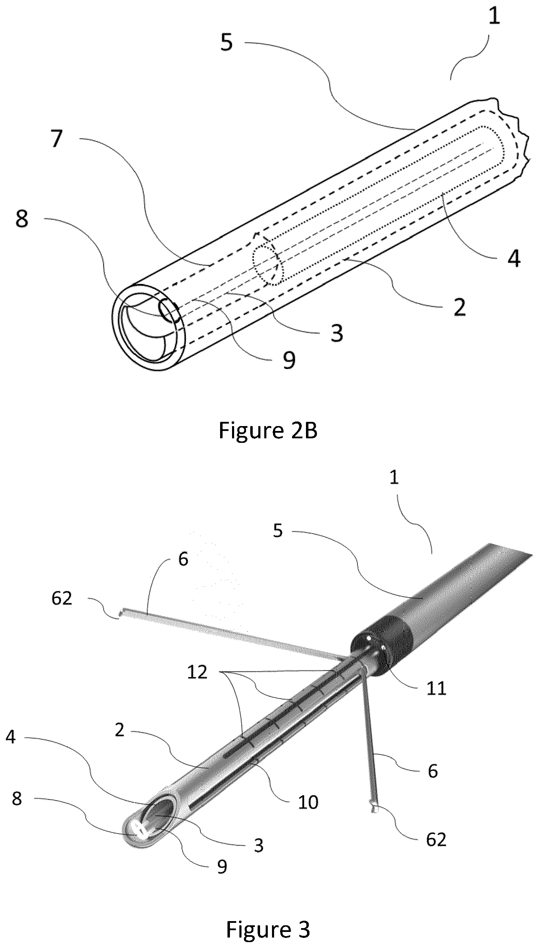

It is noted that the morcellator (1) is slidable relative to the cannula (5). Accordingly, when provided in the cannula (5) the morcellator (1) may be positioned so as to be completely housed by the cannula (5), as shown in Figures 2A and 2B. This prevents premature exposure of tissue to the morcellator (1).

As shown in the preferred embodiment of **Figure 3**, in any of the embodiments disclosed herein, one or more of the clamping elements (6) may comprise an inwardly-facing protrusion (62) in order to improve the grip with the captured tissue. The at least two clamping elements (6) may take any suitable shapes in order to bring the captured tissue in contact with the ablation element (8), such as straight, curved, concave, convex or comb-shaped clamping elements, among others. clamping elements

The at least two clamping elements (6), may be opened and closed to bring the captured tissue in contact with the ablation element (8) via any suitable user interface. The at least two clamping elements (6) may be mechanically connected to a manual user interface (not shown) that allows the surgeon to open and close them. The mechanical connection may comprise one or more shafts, cables, gears or combinations thereof, to transmit the input of the surgeon to movements of the clamping elements (6) via the mechanical connection. The manual user interface may be comprised of pulleys and/or levers and/or knobs and/or any other interface that can mechanically open and close the at least pair of clamping elements (6). Alternatively, the at least pair of clamping elements (6) may be opened and closed using one or more motors that are electrically connected to the interface system and may open and close the clamping elements (6) through a mechanical connection as aforementioned. The interface system may further comprise buttons, displays, touchscreens and other means to electrically control the one or more motors. The at least pair of clamping elements (6) may alternatively be electronically controlled through a wireless connection from the interface system. In another alternative, the at least pair of clamping elements (6) may comprise one or more spring loaded mechanisms that allows the surgeon to open and close them by interacting with the interface system. It is noted that the interface system may be located at the proximal side of the morcellator (1), or separately for the morcellator (1). Therefore, in some embodiments, the extension and retraction of the clamping elements (6) may be controlled remotely.

In any of the embodiments disclosed herein, the at least pair of clamping elements (6), extension and retraction may be controlled singularly, where all clamping elements open and close at the same time using a single actuator, or may be controlled independently or in subgroups by different actuators, so that the different clamping elements (6) open and close at different times in a desired way depending on which actuator is actuated. Optionally, clamping elements extension and retraction of the clamping elements (6) may be predefined according to a desired operation. This provides the surgeon a fine control on the extension and retraction of the clamping elements in order to bring the captured tissue in contact with the ablation element (8).

It is further noted that in any of the embodiments disclosed herein the at least pair of clamping elements (6) may be opened and closed via different mechanisms on the distal end connected to any of the interfaces aforementioned. In a preferred embodiment, the at least pair of clamping elements (6) are resiliently deformable arms having a relaxed configuration and a deformed configuration, and open when in a relaxed configuration. This may be achieved through, for example, a shape memory material such as a shape memory polymer or metal. The clamping elements (6) may be attached to the tubular body (2) by any suitable means, such as welding or adhesive. In such embodiments, when the clamping elements (6) are free from external forces such as in Figure 1A, the clamping elements (6) are naturally biased towards the open configuration. The at least pair of clamping elements (6) may be closed by translating a sheath over the clamping elements (6) to bias the clamping elements (6) into the closed configuration like that shown in Figure 1B. In some embodiments, the sheath, may be the cannula (5), so that when the elongate body (2) is wholly or partially fit inside the cannula (5) as shown in Figure 2, the clamping elements (6) are biased into the closed configuration, towards the ablation element (8). Therefore, the captured tissue is brought towards the ablation element (8) by sliding the elongate body (2) inside the cannula (5) or by sliding the cannula (5) over the elongate body (2). Alternatively, the sheath may be a separate element, in form of a sleeve slidably received in the cannula (5) between the cannula (5) and the elongate body (2) and configured to extend over the clamping elements (6) to bias them into the closed position, and retractable to release the clamping elements (6) recovering their open position. In such embodiments, movement of the clamping elements between the open and closed configuration is effected by movement of a proximal end of a sleeve or sheath at a proximal end of the morcellator by the surgeon.

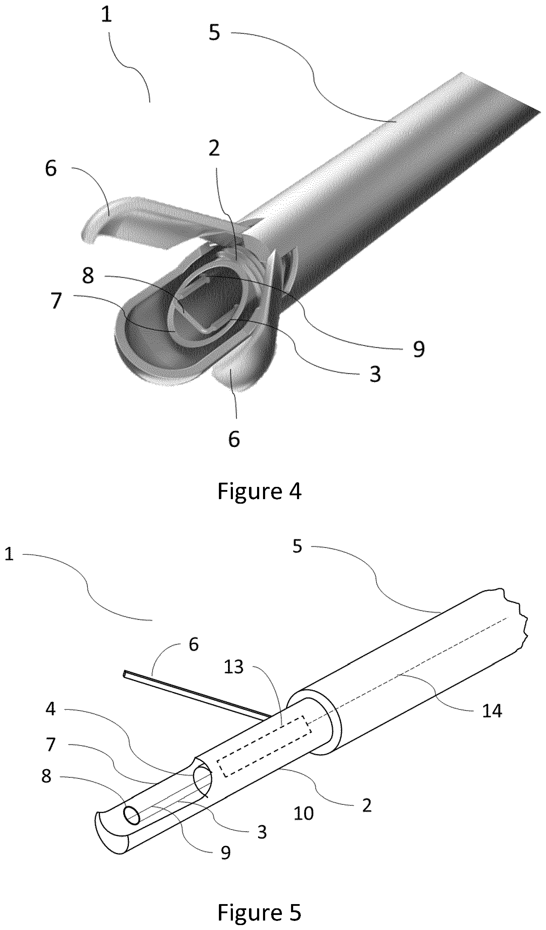

Alternatively, in another embodiment, the at least pair of clamping elements (6) comprises a magnetic material such as a ferromagnetic or ferrimagnetic material, so that the clamping elements (6) can be moved between the open configuration and the closed configuration via a repulsive and/or attractive magnetic force. In such embodiments, the morcellator (1) may comprise an electromagnet configured to be electrically actuated to attract or repel the at least pair of clamping elements (6) towards the desired open or closed configurations. Figure 5 shows a schematic diagram of one such embodiment. It is noted that one of the clamping elements (6) is omitted from the diagram for clarity but the embodiment includes at least a pair of clamping elements (6) In the illustrated embodiment, the morcellator (1) comprises at least one electromagnet (13) configured to attract and/or repel the clamping elements (6) when current is applied to the electromagnet (13). For this purpose, the electromagnet is connected to an electronic controller (not shown) through electrical connection (14), wherein the electronic controller is configured to switch on and off the electromagnet (13) to move the clamping elements (6) between the open and closed configurations.

In one embodiment, the clamping elements (6) may comprise a resiliently deformable material, for example mounted to the tubular body (2), and be naturally biased towards the open configuration, and the electromagnet (13) may be configured to exert an attractive force on the clamping elements (6) when switched on by the electronic controller. Accordingly, the clamping elements (6) move to an open configuration when the electromagnet (13) is switched off and to a closed configuration when the electromagnet (13) is switched on. The tissue can therefore be clamped and brought into contact with the ablation element (8) when the electromagnet (13) is switched on. In an alternative embodiment, the clamping the clamping elements (6) may comprise a resiliently deformable material mounted to the morcellator, for example to the tubular body (2) and be naturally biased towards the closed configuration, and the electromagnet (13) may be configured to exert a repulsive force on the clamping elements (6) when switched on by the electronic controller. Accordingly, the clamping elements (6) move to an open configuration when the electromagnet (13) is switched on and to a closed configuration when the electromagnet (13) is switched off. The tissue can therefore be clamped and brought into contact with the ablation element (8) when the electromagnet (13) is switched off. In an alternative embodiment, the clamping elements (6) are pivotally mounted to the morcellator (1), for example to the tubular body (2), and the electromagnet (13) have a reversible polarity, that is to say, the electronic controller may be configured to control the electromagnetic in an attractive mode and a repulsive mode (by e.g. providing a forward current and a reverse current to the electromagnet). Accordingly, the clamping elements (6) move to an open configuration when the electromagnet (13) is switched on in a repulsive mode and to a closed configuration when the electromagnet (13) is switched on in an attractive mode. The tissue can therefore be clamped and brought into contact with the ablation element (8) when the polarity of the electromagnet (13) is reversed by the electronic controller. It is noted that the electromagnet may be comprised in an element of the tissue morcellator (1) separate to the tubular body (2), and that more than one electromagnet (13) may be provided to control the clamping elements (6), and each electromagnet (13) may provide an attractive and/or repulsive force. The electronic controller may be controller by a user by any suitable user interface such as those disclosed herein.

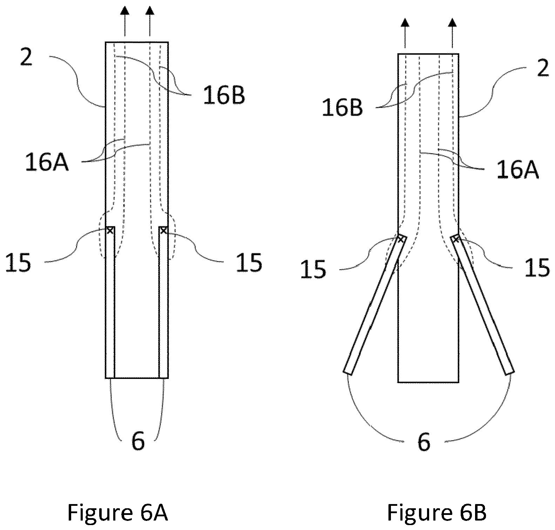

In an alternative preferred configuration, the at least pair of clamping elements (6) are pivotably mounted to the morcellator (1), and more specifically the tubular body (2) via respective hinges (15) in the proximal end of the clamping elements (6) and configured to be opened and closed through mechanical elements connected to a any suitable user interface at a proximal end of the morcellator as previously described. For example, in Figures 6A and 6B, a schematic view of the elongate body (2) with a pair of clamping elements (6) with a hinge (15) is shown in a closed (Fig. 6A) and open (Fig. 6B) configuration. Other required elements such as the ablation element and the fluid evacuation conduit are not shown for clarity purposes. The pair of clamping elements (6) are connected to a one or more actuators on the user interface via one or more wires (16) extending through the morcellator (1) from a proximal end to the distal portion of the morcellator (1), configured to open and close the clamping elements (6) when the wires are pulled and/or pushed through the pulley system in the controller at the proximal end of the tissue morcellator (1). For example, a wire (16A) may be connected to one or more clamping elements (6) so that when the wire (16A) is pulled in the proximal direction by a user using the user interface (for example by pulling a lever, rotating a wheel or actuating a motor), the clamping elements are configured into the closed configuration, while another wire (16B) may be configured connected to one or more clamping elements (6) so that when the wire (16B) is pulled, the clamping elements are moved to the open configuration or vice versa. The movement between open and closed configurations is defined by a pivoting movement of the clamping elements (6) with respect to hinges (15). It is noted that different wire (16) and pulley system combinations of push/pull designs may be designed to move the clamping elements (6) to an open or closed configuration using any suitable user interface. It is further noted that the mechanism may configure the clamping elements (6) to open and/or close with a higher or lower force than the one exerted through the interface in the controller as generally known in the art, for example by the use of transmission gears between the user interface and the clamping elements (6).

Therefore, when the at least pair of clamping elements (6) are retracted, they bring any tissue material in contact with the ablation element (8) such that the material would be cut in smaller pieces. In this way, small pieces of material can be suctioned out of the instrument through the fluid evacuation conduit (4). The fluid evacuation conduit (4) may be connected to a surgical vacuum tube, or to an external suction system of any type. The fluid evacuation conduit (4) may take several shapes in order to suction fluid from the lateral aperture. For example, the fluid evacuation conduit (4) may run longitudinally parallel to the elongate body (2) as a separate element. In some embodiments like in **Figures 1A, 1B****,** **1C, 2A****,** **2B and 3**, the fluid evacuation conduit (4) may be comprised within the elongate body (2).

In the preferred embodiment of **Figures 1A, 1B** **and** **1C**, the elongate body (2) is a tubular body comprising the fluid evacuation conduit (4), the ablation element (8) located in the elongate body (2) and exposed via an aperture (7) in the tubular body (2), wherein the aperture (7) is fluidly connected to a distal portion of the evacuation conduit (4). More preferably, the aperture comprises an aperture (7) having a lateral portion, namely a portion extending at least partially in a longitudinal direction in a sidewall of the tubular body (2), and the at least pair of clamping elements (6) are configured to move towards the lateral aperture (7) from the open configuration to the closed configuration.

It is noted that the aperture (7) may take different shapes and sizes. Also, the aperture may be partially located on the distal end of the tubular body (2) so that it has both a longitudinal and radial extent. It is also noted that in some embodiments, the tubular body (2) may comprise more than one lateral aperture (7), with different shapes and/or locations. In some embodiments, the at least pair of clamping elements (6) are pivotably mounted to the morcellator (1), preferably wherein the at least pair of clamping elements (6) are pivotably mounted to the elongate body (2).The at least pair of clamping elements (6) and the ablation element (8) being located on the same body allows for a more compact design.

In any of the embodiments disclosed herein, one or more of the at least pair of clamping elements (6) are preferably received in one or more recesses (10) of the morcellator (1) in the closed position, preferably wherein the one or more clamping elements (6) are fully received within the one or more recesses (10) in the closed position as represented in **Figure 1B****.** The recesses (10) are not shown as they are covered by the clamping elements (8). It is noted that in **Figure 1A**, only one recess (10) is shown for clarity purposes, but a second recess (10) may be provided on the hidden side of the tubular body (2). Similarly, in Figures 1C and 2B the clamping elements (6) and their respective recesses are not shown for clarity purposes. When the at least pair of clamping elements (6) are configured to be opened and closed from a recess (10) in the tubular body (2) the at least pair of clamping elements (6) are at least partially housed in the tubular body (2) surface, which allows for a smoother design of the morcellator (1) in the closed configuration and further reduces the chances of damaging healthy tissue during the operation of the morcellator (1).

In some embodiments such as those shown in **Figures 1A, 1B** **and** **1C**, the ablation element (8), comprises a plasma ablation element. The plasma ablation element may be any suitable element which provides heating of the tissue via a plasma generated by the plasma ablation element to ablate exposed tissue. Plasma ablation provides some advantages over other types of ablation. For example, it requires lower levels of energy which implies lower operating temperatures, which in turn implies less thermal spread over the tissue, therefore reducing the heating effects in the surrounding tissue. Another advantage of plasma ablation elements is that they can provide a higher cutting precision than other ablation elements, and furthermore its shape can be tuned to fit a certain ablation need.

In some embodiments such as the illustrated embodiments of Figures 1A to 5, the ablation element (8) comprises a conductive wire connected between a feed electrode (3) and a return electrode (9). It is noted that the conductive wire may take several shapes. For example, it can take a linear shape such as shown in Figure 4, a circular shape such as shown in Figure 4, a rounded shape or any other shape feasible to be achieved with a plasma means material. Moreover, the conductive wire may be angled to enhance the ablation of certain regions. For example the conductive wire of the plasma ablation element (8) of **Figures 1A, 1B****,** **1C** **and** **3** are of a circular shape and disposed in a 90° angle with respect to the elongate body (2) axis, so that in use a circular cut is produced when the ablation means (8) are longitudinally moved across the tissue. In another example, Figure 4 shows a plasma ablation element (8) with a linear shape. Operation of the ablation element (8) may be controlled via any suitable user interface operably connected to an electronic controller for a voltage or current source configured to provide an electrical current at the required frequency and voltage, the voltage or current source being connectable to the feed and return electrodes at a proximal end of the morcellator. In particular, the user interface may be operable to switch the voltage or current source on or off in order to start or stop delivery of the plasma. The electronic controller may be configured to control the voltage or current source so that it provides the electrical current intermittently in pulses, so that the plasma is provided in pulses.

In other embodiments, the ablation element (8) is a laser ablation element. For example, a laser of a determined wavelength and power may be configured to vaporize a specific tissue such as prostate tissue. In such embodiments, the ablation element may comprise a laser source at the proximal end of the morcellator (1), and a waveguide or optical fibre extending from the laser source to the distal portion of the morcellator (1), for example through a lumen in the tubular body (2). The distal tip of the waveguide or optical fibre emits the laser light received from the laser source onto the target tissue, which is ablated by the intensity of the laser light. Any laser wavelength and intensity suitable for tissue ablation may be used, depending on the desired rate of ablation.

In other alternative embodiments, the ablation element (8) is a mechanical ablation element, such as a grinder or a cutting device. For example, the ablation element (8) may comprise a pair of toothed blades or a rotary cutter configured to cut the tissue into pieces, so that the resulting pieces can be extracted through the evacuation conduit via suction for later histological analysis. The clamping elements (6) efficiently clamps tissue and brings the clamped tissue into contact with the ablation element (8), increasing the effectiveness of even mechanical ablation means. It is noted that other types of ablation elements (8) may be used and the present application is not limited to the aforementioned ablation element (8) types.

In a preferred embodiment the ablation element (8) is configured to be displaceable. More preferably, the ablation element (8) is configured to be longitudinally displaceable relative to the elongate body (2) (i.e. in the proximal and distal directions). This allows the ablation element (8) to be able to longitudinally cut the tissue independently of the movement of the morcellator (1) and therefor increases the controllability of the morcellator (1). For example, in use the morcellator (1) can be located at a fixed position with respect to the inserted length along the urethra (for example locked in position using an locking tool external to the patient), and cut the tissue without longitudinally displacing the morcellator, reducing the risk of pain and the risk of healthy tissue damage. It is noted that the ablation element (8) may be longitudinally displaceable in several ways. For example, the ablation element (8) may be mechanically connected to an user interface (not shown) that allows the surgeon to longitudinally move the ablation element (8) by interacting with an actuator on the user interface. The mechanical connection may comprise one or more shafts, cables, gears or combinations therefore, to transfer the interface actions to movements of the ablation element (8) including its longitudinal displacement. The interface may comprise of pulleys and/or levers and/or knobs or any other interface that longitudinally displace the plasma ablation element (8). Alternatively, ablation element (8) may be longitudinally displaced using one or more motors that are electronically connected to an electronic user interface and may displace the ablation element (8) through a mechanical connection as aforementioned. The interface system may further comprise buttons, displays, touchscreens or other means to electrically control the one or more motors. The ablation element (8) may alternatively be electronically controlled through a wireless connection from the user interface. It is noted that the user interface may be located at the proximal side of the morcellator (1), or separately from the morcellator (1). Therefore, in some embodiments, the longitudinal displacement of the ablation element (8) may be controlled remotely. Alternatively, the ablation element (8) may be displaced by movement of whichever proximal element is connected to the ablation element (8) (for example the return and feed electrodes or the waveguide or optical fibre).

In another preferred embodiment, the ablation element (8) is configured to be axially rotatable relative to the elongate body (2). This allows the morcellator (1) to be able to cut the tissue around the elongate body (2) longitudinal axis of rotation independently of the movement of the morcellator (1), increasing controllability of the morcellator (1). It is noted that in some embodiments, the ablation element (8) may be configured to be displaceable in more than one direction relative to the elongate body (2). For example, the ablation element may be configured to be longitudinally displaceable, and axially rotatable relative to the elongate body (2). It is further noted that the ablation element (8) may be axially rotatable in several ways as aforementioned with respect to its longitudinal displacement.

**Figures 1C** **and** **2B** show the see-through view of the morcellator (1) comprising the cannula (5) when the elongate body (2) is extended and retracted from the distal part of the cannula (5), respectively, according to one or more embodiments. It can be seen that the elongate body (2) slidably fits inside the cannula (5). The fluid evacuation conduit (4), the feed electrode (3) and the return electrode (9) are comprised withing the elongate body (2). In other embodiments, the fluid evacuation conduit (4), the feed electrode (3) and the return electrode (9) may not be comprised withing the elongate body (2). For example, the feed electrode (3) and the return electrode (9) may externally run longitudinally parallel to the elongate body (2) as separate elements of the morcellator (1).

In a preferred embodiment, the plasma ablation element (8) comprises an electrically conductive wire connected to a bipolar electrode, and namely between at least one feed electrode (3) and at least one return electrode (9). When a high frequency electrical current (for example several hundred kHz, particularly above 400 kHz) at a voltage of about 500V or more is passed through the plasma ablation element (8) via the feed and return electrodes, a plasma corona is generated at the plasma ablation element (8). More preferably, the feed electrode (3) is made of a biocompatible metal or metal alloy. Preferably the metal or metal alloy is a high temperature alloy configured to withstand the temperatures generated by the plasma ablation element (8) This allows the feed electrode (3) to be able to support high temperatures including plasma temperatures. In an even more preferred embodiment, the feed electrode (3) is made of: tungsten, tungsten-rhenium, platinum-iridium, titanium, or stainless steel. It is noted that the width of the feed electrode (3) of the ablation element (8) may have different widths depending on the application. Insulation sleeves (not shown) are placed around the feed electrode (3) in a known manner, in order to electrically isolate the feed electrode (3) and the return electrodes (9). Preferably, the morcellator (1) comprises an insulating element electrically insulating a portion of feed electrode (3) from the return electrode (9) along at least a portion of the morcellator (1), wherein the bipolar electrode is exposed at the distal portion of the elongate body (2).

In a preferred embodiment, the morcellator (1) has an atraumatic tip such as a rounded tip, preferably wherein the elongate body has a rounded cross-sectional shape. This allows for a smother displacement of the morcellator (1) through the urethra, which reduces the changes of damaging healthy tissues. As shown in Figure 4, the clamping elements (6) may comprise curved tips which are flush with the distal tip of the elongate body (2) so that when the clamping elements (6) are in the closed configuration, the elongate body (2) together with the clamping elements (6) form a rounded atraumatic tip.

In any of the embodiments disclosed herein, such as illustrated in **Figure 3**, the morcellator (1) further comprises at least imaging device (11) on its distal end configured to provide a view of the distal portion of the morcellator (1) to the surgeon. More preferably, the morcellator (1) additionally comprises one or more light sources configured to provide illuminate the area imaged by the imaging device (11). The imaging device (11) and light sources may be electrically connected to one or more controllers at a proximal end of the morcellator via electrical wires extending along the length of the morcellator (1) through the cannula (5) the controller may provide electrical power to the imaging device (11) and light sources, as well as send and receive control signals and receive imaging data from the imaging device (11). The controller may further be connectable to a display device for displaying the recorded image of the imaging device (11) in real time. This allows the surgeon to be able to see in real time the clamping elements and/or the ablation element for a better control during the ablation procedure. It is noted that the camera may be able to detect light in different frequencies including visible frequencies. Likewise, the optional light source may emit light of different frequencies including visible frequencies. Alternatively, the imaging device (11) may comprise an ultrasound device. It is noted that the imaging device (11) may be located in different positions on the distal portion of morcellator (1).

In some embodiments such as that shown in Figure 3, the morcellator (1) further comprises a set of markers (12) along the distal portion of the morcellator and in the field of view of the imaging device (11) when comprised by a camera and a light source. The markers (12) are configured to provide a reference to the surgeon. The markers (12) are disposed in a predetermined way such that they can serve as a spatial reference for the surgeon. For example, two markers (12) may be at a predetermined distance away, so that the surgeon knows at all times the scale of the environment the morcellator is in and with which it interacts. More preferably, the markers (12) are equally separated at a predetermined distance. This may allow, for example, for the surgeon to know how deep the ablation element (8) is inserted within a tissue or to indicate the size of the tissue being viewed (for example the approximate size of a tumour or other tissue type). In some embodiments, the markers are preferably perpendicular to the elongated body (2) longitudinal axis. Any number of markers, that is one or more, may be provided.

In some embodiments, such as in any of the **Figures 1A, 1B****,** **1C, 2A****,** **2B 3** **and** **4** it is disclosed a tissue morcellating system comprising a tissue morcellator (1) according to any of the previous embodiments and a cannula (5) suitable to be inserted through a patient's urethra, and sized to slidably receive the tissue morcellator. In some embodiments, the at least pair of clamping elements (6) may be pivotably mounted in the cannula (5), and optionally pivotably mounted on its inner or outer surface. In an alternative preferred embodiment such as that shown in **Figure 4****,** the at least a pair of clamping elements (6) for capturing tissue is pivotably mounted at the distal end of the cannula (5) and configured to be extended and retracted to bring the captured tissue in contact with the ablation element (8). The clamping elements (6) are configured to cover the lateral opening (7) on the elongate body (2) in their closed configuration. Preferably the cannula is a metallic tube.

## Claims

1. A tissue morcellator, comprising:
an elongate body configured to slidably fit inside a cannula,
an ablation element located at a distal portion of the elongate body,
a fluid evacuation conduit for fluidly communicating the distal portion of the elongate body to a proximal portion of the morcellator and connectable to a negative pressure source at a proximal end of the fluid evacuation conduit, and
at least a two clamping elements proximal the ablation element and movable between an open configuration and a closed configuration, the clamping elements configured clamp tissue and to bring the clamped tissue into contact with the ablation element when moved from the open configuration towards the closed configuration.

2. The tissue morcellator according to claim 1, wherein the elongate body is a tubular body comprising the fluid evacuation conduit, the ablation means located in the tubular body and exposed via an aperture in the tubular body, wherein the aperture is fluidly connected to a distal portion of the evacuation conduit.

3. The tissue morcellator according to claim 2, wherein the aperture comprises a aperture having a lateral portion in a sidewall of the tubular body, and the at least two clamping elements are configured to move towards the lateral aperture from the open configuration to the closed configuration.

4. The tissue morcellator according to any preceding claim, wherein the at least two clamping elements are pivotably mounted to the morcellator, preferably wherein the at least two clamping elements are pivotably mounted to the elongate body.

5. The tissue morcellator according to any preceding claim, wherein one or more of the clamping elements are received in one or more recesses of the morcellator in the closed position, preferably wherein the one or more clamping elements are fully received within the one or more recesses in the closed position.

6. The tissue morcellator according to any of the previous claims, wherein the ablation element comprises a plasma ablation element.

7. The tissue morcellator according to any of the previous claims, wherein the ablation element is configured to be longitudinally displaceable relative to the elongate body.

8. The tissue morcellator according to claims 6 or 7, wherein the ablation element comprises a bipolar electrode, comprising at least one feed electrode and at least one return electrode.

9. The tissue morcellator according to claim 8, wherein the feed electrode is made of a biocompatible metal or metal alloy.

10. The tissue morcellator according to claim 9, wherein the feed electrode is made of: tungsten, tungsten-rhenium, platinum-iridium, titanium, or stainless steel.

11. The tissue morcellator according to any of claims 8 to 10, further comprising an insulating element electrically insulating a portion of feed electrode from the return electrode along at least a portion of the morcellator, wherein the feed electrode and the return electrode are exposed at the distal portion of the elongate body.

12. The tissue morcellator according to any of the previous claims, wherein the elongate body has an atraumatic tip, preferably wherein the elongate body has a rounded cross-sectional shape.

13. The tissue morcellator according to any of the previous claims, further comprising the negative pressure source.

14. The tissue morcellator according to any of the previous claims, further comprising an imaging device at the distal portion of the tissue morcellator.

15. A tissue morcellating system comprising:
a tissue morcellator according to any of the previous claims, and
a cannula suitable to be inserted through a patient's urethra, and sized to slidably receive the tissue morcellator.
